# EUROPEAN PATENT SPECIFICATION

(11) **EP 4 294 395 B1**
(45) Date of publication and mention of the grant of the patent: **26.03.2025**
(21) Application number: 22713478.0
(22) Date of filing: 14.02.2022
(51) Int. Cl.: A61K 31/437, A61P 35/00, A61P 35/04, A61K 31/553, A61K 31/422

(54) **TREATMENT OF BREAST CANCER USING COMBINATION THERAPIES COMPRISING GDC-9545 AND GDC-0077**
BEHANDLUNG VON BRUSTKREBS MIT KOMBINATIONSTHERAPIEN MIT GDC-9545 UND GDC-0077
TRAITEMENT DU CANCER DU SEIN À L'AIDE DE POLYTHÉRAPIES COMPRENANT GDC-9545 ET GDC-0077

(30) Priority: 16.02.2021 US 202163149944 P
(43) Date of publication of application: 27.12.2023
(73) Proprietor: Genentech, Inc., South San Francisco, CA 94080-4990 (US)
(72) Inventor: METCALFE, Ciara, San Francisco, California 94080-4990 (US); WANG, Xiaojing, San Francisco, California 94080-4990 (US); ZHOU, Wei, San Francisco, California 94080-4990 (US)
(74) Representative: Neuhaus, Christian Michel
(86) International application number: PCT/US2022/016270
(87) International publication number: WO 2022/177844

(56) References cited:
- WO-A1-2017/216280
- WO-A1-2020/023297
- WO-A2-2020/037203
- ANONYMOUS: "A Study Evaluating the Efficacy and Safety of Multiple Treatment Combinations in Participants With Breast Cancer", CLINICALTRIALS.GOV, 17 March 2021 (2021-03-17), Internet, pages 1 - 13, XP055918964, Retrieved from the Internet <URL:https://clinicaltrials.gov/ct2/show/record/NCT04802759> [retrieved on 20220509]

## Description

### CROSS-REFERENCE TO RELATED APPLICATIONS

This non-provisional patent application claims the benefit of U.S. Provisional Patent Application No. 63/149,944 , filed 16 February 2021.

### FIELD OF THE INVENTION

Provided herein are combination therapies comprising a GDC-9545 or a pharmaceutically acceptable salt thereof) and GDC-0077 or a pharmaceutically acceptable salt thereof for the treatment of breast cancers. cancers.

Any reference in the description to methods of treatment refers to the compounds, pharmaceutical compositions and medicaments of the present invention for use in a method of treatment of the human (or animal) body by therapy (or diagnosis).

### BACKGROUND

Breast cancer is the most frequent cancer diagnosed in women, with an estimated global incidence of 2.1 million new cases reported in 2018 (Bray et al. CA Canver J Clin 2018;68:394-424). Breast cancer accounts for approximately 12% (approximately 627,000 cases) of all cancer deaths. Breast cancer mortality rates differ by geographical region, with more favorable survival rates observed in more developed regions of the world (Id).

Initial treatment for breast cancer is often guided by the presence of molecular markers found on breast cancer cells. These markers are used to identify breast cancer subtypes and to assist in the development of treatments based on presence of tumor hormone receptor content (estrogen receptor [ER]/progesterone receptor [PR]). Hormone receptor-positive (HR+) breast cancers with estrogen receptor-alpha (ER-α)-expression constitute 70% of all invasive breast cancers. PR expression in the tumor is another marker of ER-α signaling. Endocrine agents are the standard-of-care treatment used to downregulate ER signaling for HR+ breast cancers.

Human epidermal growth factor receptor 2 (HER2) is a transmembrane receptor tyrosine kinase that is amplified or overexpressed in 20% of breast cancers. HER2-positive breast cancer treatment regimens include HER2-directed therapies (anti-HER2 antibodies and tyrosine kinase inhibitors).

Not all HR+ breast cancers respond optimally to ET. Mechanisms that can lead to primary and/or secondary hormonal resistance in HR+ breast cancer include a decrease or loss of hormone receptor expression or an upregulation of growth factor signaling pathways, such as the epidermal growth factor receptor or HER2, the MAPK, or the PI3K/Akt/mTOR pathways (Johnston et al. 2009; Musgrove and Sutherland 2009). Recently, mutations in the gene that encodes estrogen receptor (*ESR1*) have been identified in metastatic ER-positive (ER+) tumors and are associated with resistance to anti-estrogen therapies (Robinson et al. 2013; Toy et al. 2013; Jeselsohn et al. 2014).

Accordingly, there is a pressing need for clinically active agents for treatment of relapsed or resistant ER-positive breast cancer.

WO 2017/216280 discloses tetrahydro-pyrido[3,4-b]indol-1-yl compounds including giredestrant optionally in combination with other anti-cancer drugs

WO 2020/023297 refers to a treatment of breast cancer with the PI3K inhibitor GDC-0077 and metformin.

WO 2020/037203 reports on a method of identifying and treating a subject with breast cancer with GDC-9545.

### SUMMARY

Provided herein are solutions to the problems above and other problems in the art.

The present embodiments can be understood more fully by reference to the detailed description and examples, which are intended to exemplify non-limiting embodiments.

### BRIEF DESCRIPTION OF THE FIGURES

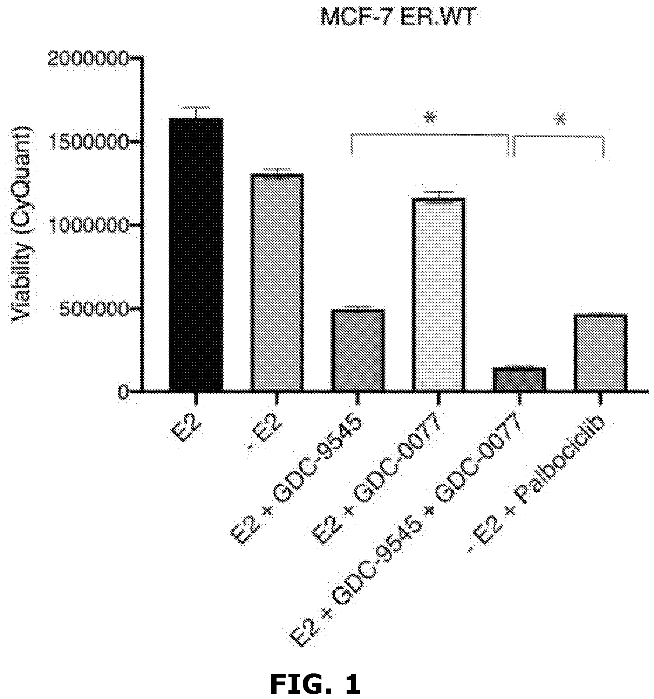

FIG. 1 depicts the anti-proliferative effect of endocrine suppression by E2 withdrawal or GDC-9545-treatment, either alone or in combination with PI3K or CDK4/6 inhibition in MCF-7 cells harboring wildtype ESR1. Cells were treated as shown for 6 to 8 days, and relative viability was assessed. * p < 0.0001, parametric t-test.

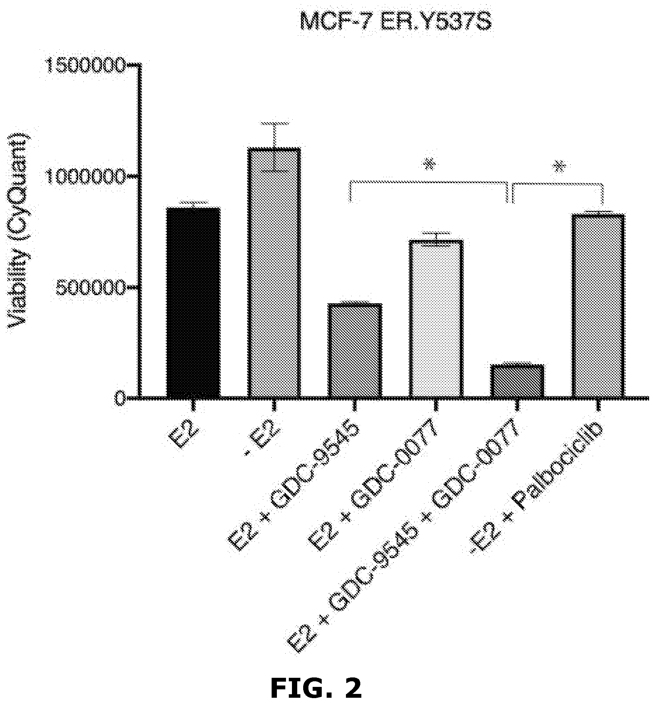

FIG. 2 depicts the anti-proliferative effect of endocrine suppression by E2 withdrawal or GDC-9545-treatment, either alone or in combination with PI3K or CDK4/6 inhibition in MCF-7 cells harboring mutant (Y537S) ESR1. Cells were treated as shown for 6 to 8 days, and relative viability was assessed. * p < 0.0001, parametric t-test.

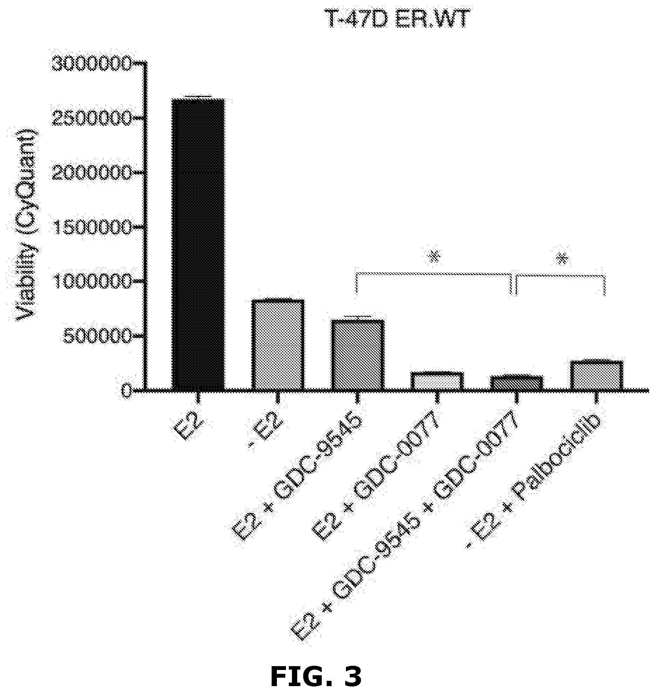

FIG. 3 depicts the anti-proliferative effect of endocrine suppression by E2 withdrawal or GDC-9545-treatment, either alone or in combination with PI3K or CDK4/6 inhibition in T-47D cells harboring wildtype ESR1. Cells were treated as shown for 6 to 8 days, and relative viability was assessed. * p < 0.0001, parametric t-test.

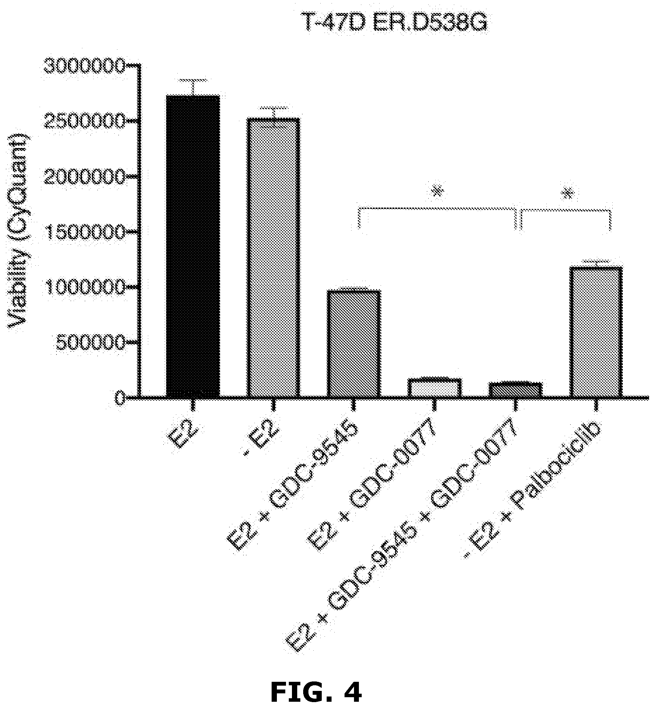

FIG. 4 depicts shows the anti-proliferative effect of endocrine suppression by E2 withdrawal or GDC-9545-treatment, either alone or in combination with PI3K or CDK4/6 inhibition in T-47D cells harboring mutant (D538G) ESR1. Cells were treated as shown for 6 to 8 days, and relative viability was assessed. * p < 0.0001, parametric t-test.

### DETAILED DESCRIPTION

Unless defined otherwise, all technical and scientific terms used herein have the same meaning as commonly understood by those of ordinary skill in the art to which the invention belongs. See, e.g., Singleton et al., DICTIONARY OF MICROBIOLOGY AND MOLECULAR BIOLOGY 2nd ed., J. Wiley & Sons (New York, NY 1994); Sambrook et al., MOLECULAR CLONING, A LABORATORY MANUAL, Cold Springs Harbor Press (Cold Springs Harbor, NY 1989). Any methods, devices and materials similar or equivalent to those described herein can be used in the practice of this invention.

The following definitions are provided to facilitate understanding of certain terms used frequently herein and are not meant to limit the scope of the present disclosure.

As used herein, and unless otherwise specified, the terms "about" and "approximately," when referring to doses, amounts, or weight percents of ingredients of a composition or a dosage form, mean a dose, amount, or weight percent that is recognized by one of ordinary skill in the art to provide a pharmacological effect equivalent to that obtained from the specified dose, amount, or weight percent. The equivalent dose, amount, or weight percent can be within 30%, 20%, 15%, 10%, 5%, 1%, or less of the specified dose, amount, or weight percent.

"GDC-9545" refers to a compound having the structure: 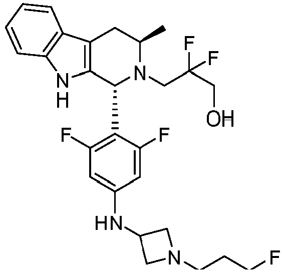 having the chemical name 3-((1R,3R)-1-(2,6-difluoro-4-((1-(3-fluoropropyl)azetidin-3-yl)amino)phenyl)-3-methyl-1,3,4,9-tetrahydro-2H-pyrido[3,4-b]indol-2-yl)-2,2-difluoropropan-1-ol, including a pharmaceutically acceptable salt thereof. In one embodiment, GDC-9545 is a tartrate salt. "GDC-9545" as used herein refers to free base and pharmaceutically acceptable salts of GDC-9545 including a tartrate salt thereof. GDC-9545 is also known as giredestrant.

"GDC-0077" refers to a compound having the structure: 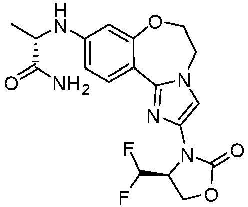 having the chemical name (2S)-2-[[2-[(4S)-4-(Difluoromethyl)-2-oxo-3-oxazolidinyl]-5,6-dihydroimidazo[1,2-d][1,4]benzoxazepin-9-yl]amino]propanamide, including a pharmaceutically acceptable salt thereof. In one embodiment, GDC-0077 is administered as free base. "GDC-0077" as used herein refers to free base and pharmaceutically acceptable salts of GDC-0077. GDC-0077 is also known as Inavolisib.

The term "package insert" is used to refer to instructions customarily included in commercial packages of therapeutic products, that contain information about the indications, usage, dosage, administration, contraindications and/or warnings concerning the use of such therapeutic products.

"Overall survival" or "OS" refers to the time from enrollment to death from any cause.

"Objective Response" refers to a complete response or partial response, as determined by an investigator according to RECIST v1.1.

"Objective response rate" or "ORR" refers the percentage of patients with a confirmed complete response or partial response on two consecutive occasions ≥ 4 weeks apart, as determined by the investigator according to RECIST v1.1.

"Time to progression" or "TTP" refers to the time from randomization until objective tumor progression.

"Duration of response" or "DOR" refers to the time from the first occurrence of a documented objective response to disease progression, as determined by the investigator according to RECIST v1.1, or death from any cause, whichever occurs first.

"Progression free survival" or "PFS" refers to the time from enrollment to the date of the first recorded occurrence of disease progression, as determined by the investigator using RECIST v1.1 or death from any cause, whichever occurs first.

"Disease Control Rate" or "DCR" refers to the proportion of patients with stable disease for at least 12 weeks or a CR or PR as determined by the investigator using RECIST v1.1.

"Clinical benefit rate" or "CBR" refers to the percentage of patients with stable disease for at least 24 weeks or with confirmed complete or partial response, as determined by the investigator according to RECIST v1.1.

"Complete response" or "CR" refers to the disappearance of all target lesions and non-target lesions and (if applicable) normalization of tumor marker level.

"Partial response" or "non-CR/Non-PD" refers to persistence of one or more non-target lesions and/or (if applicable) maintenance of tumor marker level above the normal limits. A PR can also refer to ≥ 30% decrease in sum of diameters of target lesions, in the absence of CR, new lesions, and unequivocal progression in non-target lesions.

"Progressive disease" or "PD" refers to ≥ 20% increase in sum of diameters of target lesions, unequivocal progression in non-target lesions, and/or appearance of new lesions.

"Stable disease" or "SD" refers to neither sufficient shrinkage to qualify for CR or PR nor sufficient increase growth of tumor to qualify for PD.

The term "locally advanced breast cancer" refers to cancer that has spread from where it started in the breast to nearby tissue or lymph nodes, but not to other parts of the body.

The term "metastatic breast cancer" refers to cancer that has spread from the breast to other parts of the body, such as the bones, liver, lungs, or brain. Metastatic breast cancer may also be referred to as stage IV breast cancer.

The term "treatment" refers to clinical intervention designed to alter the natural course of the patient or cell being treated during the course of clinical pathology. Desirable effects of treatment include decreasing the rate of disease progression, ameliorating or palliating the disease state, and remission or improved prognosis. For example, a patient is successfully "treated" if one or more symptoms associated with a breast cancer described herein are mitigated or eliminated, including, but are not limited to, reducing the proliferation of (or destroying) cancerous cells, decreasing symptoms resulting from the disease, increasing the quality of life of those suffering from the disease, decreasing the dose of other medications required to treat the disease, and/or prolonging survival of patients.

The term "delaying progression" of a disease refers to deferring, hindering, slowing, retarding, stabilizing, and/or postponing development of a breast cancer described herein. This delay can be of varying lengths of time, depending on the history of the cancer and/or patient being treated. As is evident to one skilled in the art, a sufficient or significant delay can, in effect, encompass prevention, in that the patient does not develop cancer.

An "effective amount" is at least the minimum amount required to effect a measurable improvement or prevention of a breast cancer described herein. An effective amount herein may vary according to factors such as the disease state, age, sex, and weight of the patient, and the ability of the agent to elicit a desired response in the patient. An effective amount is also one in which any toxic or detrimental effects of the treatment are outweighed by the therapeutically beneficial effects. Beneficial or desired results include results such as eliminating or reducing the risk, lessening the severity, delaying the onset of the disease (including biochemical, histological and/or behavioral symptoms of the disease, its complications and intermediate pathological phenotypes presenting during development of the disease), decreasing one or more symptoms resulting from the disease, increasing the quality of life of those suffering from the disease, decreasing the dose of other medications required to treat the disease, enhancing effect of another medication such as via targeting, delaying the progression of the disease, and/or prolonging survival. In some embodiments, an effective amount of the drug may have the effect in reducing the number of cancer cells; reducing the tumor size; inhibiting (i.e., slow or stop) cancer cell infiltration into peripheral organs; inhibit (i.e., slow or stop) tumor metastasis; inhibiting (i.e., slow or stop) tumor growth; and/or relieving one or more of the symptoms associated with the disorder. An effective amount can be administered in one or more administrations. An effective amount of drug, compound, pharmaceutical composition, or combination therapy described herein can be an amount sufficient to accomplish therapeutic treatment either directly or indirectly. As is understood in the clinical context, an effective amount of a drug, compound, or pharmaceutical composition may or may not be achieved in conjunction with another drug, compound, or pharmaceutical composition, or combination therapy. Thus, an "effective amount" may be considered in the context of administering one or more therapeutic agents, and a single agent may be considered to be given in an effective amount if, in conjunction with one or more other agents, a desirable result may be or is achieved.

An "E2-repressed score" as used herein, refers to a numerical value that reflects an aggregated expression level of a predetermined set of genes whose repression is reflective of estrogen receptor (ER) pathway activity.

An "E2-induced score" as used herein, refers to a numerical value that reflects an aggregated expression level of a predetermined set of genes whose induction is reflective of estrogen receptor (ER) pathway activity.

An "ER pathway activity score" as used herein, refers to a numerical value that reflects mathematical difference between the E2-induced score and the E2-repressed score.

An "administration period" or "cycle" refers to a period of time comprising administration of one or more agents described herein (i.e. GDC-9545 or a pharmaceutically acceptable salt thereof or GDC-0077 or a pharmaceutically acceptable salt thereof) and an optional period of time comprising no administration of one or more of the agents described herein. For example, a cycle can be 28 days in total length and include administration of one or more agents for 21 days and a rest period of 7 days. A "rest period" refers to a period of time where at least one of the agents described herein (e.g. GDC-9545 or a pharmaceutically acceptable salt thereof or GDC-0077 or a pharmaceutically acceptable salt thereof) are not administered. In one embodiment, a rest period refers to a period of time where none of the agents described herein (e.g. GDC-9545 or a pharmaceutically acceptable salt thereof or GDC-0077 or a pharmaceutically acceptable salt thereof) are administered. A rest period as provided herein can in some instances include administration of another agent that is not GDC-9545 or a pharmaceutically acceptable salt thereof or GDC-0077 or a pharmaceutically acceptable salt thereof. In such instances, administration of another agent during a rest period should not interfere or detriment administration of an agent described herein.

A "dosing regimen" refers to a period of administration of the agents described herein comprising one or more cycles, where each cycle can include administration of the agents described herein at different times or in different amounts.

"QD" refers to administration of a compound once daily.

"PO" refers to oral administration of an agent described herein.

A graded adverse event refers to the severity grading scale as established for by NCI CTCAE. In one embodiment, the adverse event is graded in accordance with the table below.

| Grade | Severity |
|---|---|
| 1 | Mild; asymptomatic or mild symptoms; clinical or diagnostic observations only; or intervention not indicated |
| 2 | Moderate; minimal, local, or non-invasive intervention indicated; or limiting age-appropriate instrumental activities of daily living ^{a} |
| 3 | Severe or medically significant, but not immediately life-threatening; hospitalization or prolongation of hospitalization indicated; disabling; or limiting self-care activities of daily living ^{b, c} |
| 4 | Life-threatening consequences or urgent intervention indicated ^{d} |
| 5 | Death related to adverse event ^{d} |

### Combination Therapies

Provided herein are combination therapies comprising GDC-9545 or a pharmaceutically acceptable salt thereof (e.g. GDC-9545·tartrate) and GDC-0077 or a pharmaceutically acceptable salt thereof. In one embodiment, the combination therapies described herein are useful in the treatment of certain types of breast cancer as described herein. In one aspect provided herein is a combination therapy comprising GDC-9545 or a pharmaceutically acceptable salt thereof administered QD on days 1-28 of a first 28-day cycle and GDC-0077 or a pharmaceutically acceptable salt thereof administered QD on days 1-28 of the first 28-day cycle.

In one embodiment of the combination therapy described herein GDC-9545 or a pharmaceutically acceptable salt thereof is administered as a fixed dose QD administration. In one embodiment, the administration is oral (PO), where GDC-9545 or a pharmaceutically acceptable salt thereof is formulated as a tablet or capsule. In one embodiment, GDC-9545 or a pharmaceutically acceptable salt thereof is administered at an amount of about 1mg-100mg, 1mg-50mg, 1mg-30mg, 10mg-100mg, 10mg-50mg, or 10mg-30mg QD. In another embodiment, GDC-9545 or a pharmaceutically acceptable salt thereof is adminsitered at an amount of about 1, 5, 10, 15, 20, 25, 30, 50, or 100 mg. In still another embodiment, GDC-9545 or a pharmaceutically acceptable salt thereof is administered at an amount of about 10, 30, 50, or 100 mg. In still another embodiment, GDC-9545 or a pharmaceutically acceptable salt thereof is administered at an amount of 30 mg.

In one embodiment of the combination therapy described herein, GDC-0077 is administered at an amount of 9 mg. Such administration can be in a single dose (i.e. a single or multiple pills). In one embodiment, the dose of GDC-0077 is reduced to 6 mg or 3 mg when a patient described herein experiences an adverse event. GDC-0077 can be administered PO QD as described herein. In some embodiments, GDC-0077 is administered as described herein in one or more oral tablets. In one preferred embodiment, GDC-0077 is administered at an amount of 9 mg in an oral tablet.

The combination therapies described herein can be provided as a kit comprising one or more of the agents for administration. In one embodiment, the kit includes GDC-9545 or a pharmaceutically acceptable salt thereof for administration in combination with GDC-0077 as described herein. In another embodiment, the kit includes GDC-9545 or a pharmaceutically acceptable salt thereof packaged together with GDC-0077, where the kit comprises separate formulated dosages of each agent. In still another embodiment, the kit includes GDC-9545 or a pharmaceutically acceptable salt thereof co-formulated with GDC-0077.

In one embodiment, the agents of the combination therapy described herein are supplied in a kit in a form ready for administration or, for example, as a ready-to-take oral tablet/capsule. Kits described herein can include instructions such as package inserts. In one embodiment, the instructions are package inserts - one for each agent in the kit.

Further provided are kits for carrying out the methods detailed herein, which comprise a combination therapy described herein and instructions for use in the treatment of breast cancer as described herein.

In one embodiment, the combination therapies described herein can be used for treating estrogen receptor-postitive (ER+), human epidermal growth factor receptor 2-negative (HER2-) breast cancer. In another embodiment, the combination therapies described herein can be used for treating ER+, HER2- locally advanced breast cancer (laBC) or ER+, HER2- metastatic breast cancer (mBC). In one such embodiment, the combination therapies described herein can be used for treating ER+, HER2- laBC. In one such embodiment, the combination therapies described herein can be used for treating ER+, HER2- mBC.

### Methods of Treating

Provided herein are methods of treating ER+, HER2- laBC or mBC in a patient having such a cancer. In one aspect provided herein is a method (G1) of treating laBC or mBC as described herein in a patient having such a cancer, where the method comprises administering to the patient a combination therapy comprising GDC-9545 or a pharmaceutically acceptable salt thereof and GDC-0077. In one embodiment of the method (G1) provided herein, the method is used for treating laBC. In another embodiment of the method (G1) provided herein, the method is used for treating mBC.

Further provided herein is a method (G2) treating laBC or mBC as described herein in a patient having such a cancer, where the method comprises administering to the patient a combination therapy as described herein comprising a dosing regimen comprising: (i) administering GDC-9545 or a pharmaceutically acceptable salt thereof QD on days 1-28 of a first 28-day cycle; and (ii) administering GDC-0077 QD on days 1-28 of the first 28-day cycle. In one embodiment of the method (G2) provided herein, the method is used for treating laBC. In another embodiment of the method (G2) provided herein, the method is used for treating mBC.

In one embodiment of the method of G1 or G2, GDC-9545 or a pharmaceutically acceptable salt thereof is administered as a fixed dose QD administration. In one embodiment, the administration is oral (PO), where GDC-9545 or a pharmaceutically acceptable salt thereof is formulated as a tablet or capsule. In one embodiment, GDC-9545 or a pharmaceutically acceptable salt thereof is administered at an amount of about 1mg-100mg, 1mg-50mg, 1mg-30mg, 10mg-100mg, 10mg-50mg, or 10mg-30mg QD. In another embodiment, GDC-9545 or a pharmaceutically acceptable salt thereof is adminsitered at an amount of about 1, 5, 10, 15, 20, 25, 30, 50, or 100 mg. In still another embodiment, GDC-9545 or a pharmaceutically acceptable salt thereof is administered at an amount of about 10, 30, 50, or 100 mg. In still another embodiment, GDC-9545 or a pharmaceutically acceptable salt thereof is administered at an amount of about 30 mg.

In one embodiment of method G1 or G2 described herein, GDC-0077 is administered at an amount of 9 mg. Such administration can be in a single dose (i.e. a single or multiple pills). In one embodiment, the dose of GDC-0077 is reduced to 6 mg or 3 mg when a patient described herein experiences an adverse event. GDC-0077 can be administered PO QD as described herein. In some embodiments, GDC-0077 is administered as described herein in one or more oral tablets. In one preferred embodiment, GDC-0077 is administered at an amount of 9 mg in an oral tablet.

Still further provided herein is a method (G3) of treating laBC or mBC in a patient having such a cancer where the method comprises administering to the patient a combination therapy described herein comprising a dosing regimen comprising: (i) administering 30 mg GDC-9545 or a pharmaceutically acceptable salt thereof QD on days 1-28 of a first 28-day cycle; and (ii) administering 9 mg GDC-0077 QD on days 1-28 of the first 28-day cycle. In one such embodiment, the dosing regimen includes 2 or more cycles as described herein. In one embodiment of the method (G3) provided herein, the method is used for treating laBC. In another embodiment of the method (G3) provided herein, the method is used for treating mBC.

In one embodiment of the methods G1, G2, and G3, the cancer is inoperable locally advanced (laBC) or metastatic ER+ breast cancer (mBC).

In one embodiment of the methods G1, G2, and G3, the combination of GDC-9545 or a pharmaceutically acceptable salt thereof and GDC-0077 does not require coadministration (treatment) with gonadotropin releasing hormone (GnRH) agonist.

In one embodiment of the methods G1, G2, and G3, the administered amount of GDC-0077 can be reduced. In one such embodiment, the dose of GDC-0077 is reduced by 3 mg in a maximum of 2 total reductions (i.e. a reduction to 6 mg QD or to 3 mg QD). In one embodiment of the methods G1, G2, and G3, administration of one agent in the combination therapy (GDC-9454 or a pharmaceutically acceptable salt thereof or GDC-0077) can be interrupted by a maximum of 28 days. In one embodiment of the methods G1, G2, and G3, the dose of GDC-9545 is not reduced.

The methods G1, G2, and G3 of treating breast cancer as provided herein can include administration of a combination therapy described herein as part of a dosing regimen. In one embodiment, the dosing regimen comprises one or more cycles. In another embodiment, the dosing regimen comprises at least 2 cycles. In another aspect provided herein is the dosing regimen comprises 2, 3, 4, 5, 6, 7, 8, 9, 10, 11, 12, 13, 14, 15, 16, 17, 18, 19, 20, 21, 22, 23, 24, 30, 36, 42, 48, 54, 60, 66, or 72 cycles. In still another embodiment, dosing regimen comprises about 2-72, 2-66, 2-60, 2-54, 2-48, 2-42, 2-36, 2-30, 2-24, 2-18, or 2-12 cycles. In one embodiment, the dosing regimen includes administration of a combination therapy as described herein in any number of cycles until the desired response (e.g. OR, PFS, OS, ORR, DOR, CBR) reaches a desired outcome (e.g. increase in OR, PFS, OS, ORR, DOR, CBR compared to a control described herein). In another embodiment, the dosing regimen includes administration of a combination therapy as described herein in any number of cycles until toxicity develops or the patient otherwise experiences one or more adverse events (AEs) that prevents further administration. In still another embodiment, the dosing regimen includes administration of a combination therapy as described herein in any number of cycles until disease progression.

In one embodiment of the methods described herein, the patient is a postmenopausal woman. In one such embodiment, the patient is (i) ≥ 60 years; (ii) Age < 60 years and has ≥ 12 months of amenorrhea plus follicle-stimulating hormone (FSH) and plasma estradiol levels within postmenopausal range by local laboratory assessment, in the absence of oral contraceptive pills, hormone replacement therapy, or gonadotropin-releasing hormone agonist or antagonist; or (iii) has had documented bilateral oophorectomy.

In another embodiment of the methods described herein, the patient is a premenopausal or perimenopausal (i.e., not postmenopausal) woman. In one such embodiment, the patient is treated with LHRH agonist in combination with a combination therapy described herein. The LHRH agonist therapy may be initiated 28 days prior to Day 1 of Cycle 1. In one embodiment, the LHRH agonist is administered on Day 1 of each cycle.

In another embodiment of the methods described herein, the patient is a man. In one such embodiment, the patient is treated with a LHRH agonist in combination with a combination therapy described herein.

In one embodiment of the methods described herein, a patient described herein has been tested for the presence of estrogen receptor, prostaglandin receptor, or Ki67. In one embodiment of the methods described herein, a patient described herein has a documented ER-positive tumor according to American Society of Clinical Oncology/College of American Pathologists guidelines. In one such embodiment, a patient described herein has a documented HER2-negative tumor.

In one embodiment of the methods described herein, a patient described herein is treatment naive. In one such embodiment, a patient described herein has not received prior chemotherapy before administration of a combination therapy described herein. In another embodiment of the methods described herein, a patient described herein has not been previously treated with an aromatase inhibitor or a CDK4/6 inhibitor (e.g. palbociclib, abemaciclib, or ribociclib) or a combination thereof. In one such embodiment, the aromatase inhibitor is anastrozole, exemestane, or letrozole. In one embodiment, a patient described herein has not been previously treated with either letrozole or palbociclib or a combination thereof. In still another embodiment of the methods described herein, a patient described herein has not received surgery, chemotherapy, or radiotherapy at least 14 days before administration of the combination therapy described herein. In still another embodiment of the methods described herein, a patient described herein has not been previously treated with a SERD (e.g. fulvestrant) or with tamoxifen. In another embodiment of the methods described herein, a patient may have been previously treated with tamoxifen, provided that the patient did not exhibit disease recurrence within the first 24 months of treatment with tamoxifen.

In one embodiment of the methods described herein, a patient has been treated with one or more cancer therapies before administration of a combination therapy described herein. In one embodiment, a patient described herein has been previoulsy treated with tamoxifen. In another embodiment, a patient described herein has been previously treated with a PI3K inhibitor or a mTOR inhibitor prior to administration of the combination therapy. In another embodiment, a patient described herein has been previously treated with fulvestrant.

In one embodiment of the methods described herein, a patient has breast cancer described herein that is resistant to one or more cancer therapies. In one embodiment of the methods described herein, resistance to cancer therapy includes recurrence of cancer or refractory cancer. Recurrence may refer to the reappearance of cancer, in the original site or a new site, after treatment. In one embodiment of the methods described herein, resistance to a cancer therapy includes progression of the cancer during treatment with the anti-cancer therapy. In some embodiments of the methods described herein, resistance to a cancer therapy includes cancer that does not response to treatment. The cancer may be resistant at the beginning of treatment or it may become resistant during treatment.

Systemic chemotherapy is considered as one standard of care (SOC) for patients with mBC, although no standard regimen or sequence exists. In one embodiment of the methods described herein, a patient described herein has been previously treated with one or more of the therapies selected from the group consisting of anastrozole, letrozole, exemestane, everolimus, palbociclib and letrozole, fulvestrant, tamoxifen, toremifene, megestrol acetate, fluoxemesterone, ethinyl estradiol doxorubicin, pegylated liposomal doxorubicin, epirubicin, cyclophosphamide, docetaxel, paclitaxel, albumin-bound paclitaxel, methotrexate, 5-fluorouracil (5-FU), methotrexate and 5-fluorouracil (5-FU), carboplatin, cisplatin, capecitabine, gemcitabine, vinorelbine, eribulin, ixabepilone, trastuzumab and pertuzumab, or a combination thereof prior to administration of a combination therapy described herein.

In one embodiment of the methods described herein, a patient described herein can have laBC or mBC as described herein that is resistant to one or more of the single agent therapies selected from the group consisting of anastrozole, letrozole, exemestane, everolimus, palbociclib and letrozole, fulvestrant, tamoxifen, toremifene, megestrol acetate, fluoxemesterone, ethinyl estradiol doxorubicin, pegylated liposomal doxorubicin, epirubicin, cyclophosphamide, docetaxel, paclitaxel, albumin-bound paclitaxel, methotrexate, 5-fluorouracil (5-FU), methotrexate and 5-fluorouracil (5-FU), carboplatin, cisplatin, capecitabine, gemcitabine, vinorelbine, eribulin, ixabepilone, trastuzumab and pertuzumab, or a combination thereof.

In one embodiment of the methods described herein, a patient described herein may have undergone surgical treatment such as, for example, surgery that is breast-conserving (i.e., a lumpectomy, which focuses on removing the primary tumor with a margin), or more extensive (i.e., mastectomy, which aims for complete removal of all of the breast tissue) prior to administration of a combination therapy described herein. In another embodiment of the methods described herein, a patient described herein may undergo surgical treatment following treatment with a combination therapy described herein.

Radiation therapy is also administered post-surgery to the breast/chest wall and/or regional lymph nodes, with the goal of killing microscopic cancer cells left post-surgery. In the case of a breast conserving surgery, radiation is administered to the remaining breast tissue and sometimes to the regional lymph nodes (including axillary lymph nodes). In the case of a mastectomy, radiation may still be administered if factors that predict higher risk of local recurrence are present. In some embodiments of the methods provided herein a patient described herein may have received radiation therapy prior to administration of a combination therapy described herein. In other embodiments of the methods provided herein a patient described herein may have receive radiation therapy following administration of a combination therapy described herein.

In some embodiments of the methods described herein, a patient described herein does not have a history of other malignancy within 5 years prior to administration of a combination therapy described herein. In some embodiments of the methods described herein, a patient described herein does not have active inflammatory bowel disease, chronic diarrhea, short bowel syndrome, or major upper gastrointestinal surgery including gastric resection. In some embodiments of the methods described herein, a patient described herein does not have cardiac disease or cardiac dysfunction.

In one embodiment of the methods described herein, treatment with a combination therapy according to the methods provided herein increases a patient's OS comparable to a control (e.g. non-treatment, standard of care (SOC) treatment, or treatment with one agent described herein (e.g. GDC-9545 or GDC-0077) alone). In one embodiment of the methods described herein, treatment with a combination therapy according to the methods provided herein increases a patient's OS comparable to a control (e.g. non-treatment, SOC treatment, treatment with one agent described herein (e.g. GDC-9545 or GDC-0077) alone) by 1, 2, 3, 4, 5, 6, 7, 8, 9, 10, 11, 12, 14, 16, 18, 20, 24 or more months comparable to the control.

In one embodiment of the methods described herein, treatment with a combination therapy according to the methods provided herein increases the patient's amount of ORR. In one such embodiment, treatment with a combination therapy according to the methods provided herein results in more patients having a complete response (CR) or partial response (PR) than compared to a control. In another embodiment of the methods described herein, the TTP is increased in a patient following treatment with a combination therapy according to the methods provided herein. In still another embodiment of the methods described herein, duration of response to the combination therapy is increased compared to a control (e.g. non-treatment, SOC treatment, or treatment with one agent described herein (e.g. GDC-9545 or GDC-0077) alone). In one such embodiment, the duration of response is increased by at least 1-3, 2-6, 3-8, 4-10, 5-12, 6-15, 8-20, or 1-24 months. In still another embodiment of the methods described herein, a patient described herein has increased clinical benefit rate compared to a control (e.g. non-treatment, SOC treatment, treatment with GDC-9545 alone, or treatment with GDC-0077 alone). In still another embodiment of the methods described herein, a patient has increased progression-free survival compared to a control (e.g. non-treatment, SOC treatment, treatment with GDC-9545 alone, or treatment with GDC-0077 alone).

In one embodiment of the methods provided herein a patient is diagnosed having a CR following treatment with a combination therapy according to the methods provided herein. In one embodiment of the methods provided herein a patient is diagnosed having a PR following treatment with a combination therapy according to the methods provided herein. In one embodiment of the methods provided herein a patient is diagnosed having SD following treatment with a combination therapy according to the methods provided herein.

Further provided herein is the use (U1) of a combination therapy described herein comprising GDC-9545 or a pharmaceutically acceptable salt thereof and GDC-0077 or a pharmaceutically acceptable salt thereof for the treatment of laBC or mBC as described herein. In one embodiment, is a use (U2) of a combination therapy described herein comprising GDC-9545 or a pharmaceutically acceptable salt thereof and GDC-0077 or a pharmaceutically acceptable salt thereof for the treatment of laBC or mBC as described herein. In one embodiment, is a use (U3) of a combination therapy described herein comprising GDC-9545 or a pharmaceutically acceptable salt thereof and GDC-0077 or a pharmaceutically acceptable salt thereof herein for the treatment of laBC or mBC as described herein.

Further provided herein is the use (GU1) of a combination therapy described herein comprising GDC-9545 or a pharmaceutically acceptable salt thereof and GDC-0077 as described herein for the treatment of mBC as described herein. Still further provided herein is the use (GU2) of a combination therapy described herein comprising GDC-9545 or a pharmaceutically acceptable salt thereof and GDC-0077 as described herein for the treatment of laBC as described herein.

Further provided herein is the use (GU3) of a combination therapy described herein comprising GDC-9545 or a pharmaceutically acceptable salt thereof and GDC-0077 as described herein for the treatment of laBC or mBC as described herein comprising a dosing regimen comprising: (i) administering GDC-9545 or a pharmaceutically acceptable salt thereof QD on days 1-28 of a first 28-day cycle; and (ii) administering GDC-0077 QD on days 1-28 of the first 28-day cycle. In one embodiment of the use (GU3) provided herein, the combination therapy is for the treatment of laBC. In another embodiment of the use (GU3) provided herein, the combination therapy is for the treatment of mBC.

Further provided herein is the use (GU4) of a combination therapy described herein comprising GDC-9545 or a pharmaceutically acceptable salt thereof and GDC-0077 as described herein for the treatment of laBC or mBC as described herein comprising a dosing regimen comprising: (i) administering 30 mg GDC-9545 or a pharmaceutically acceptable salt thereof QD on days 1-28 of a first 28-day cycle; and (ii) administering 9 mg GDC-0077 QD on days 1-28 of the first 28-day cycle. In one such embodiment, the dosing regimen includes 2 or more cycles as described herein. In one embodiment of the use (GU4) provided herein, the combination therapy is for the treatment of laBC. In another embodiment of the use (GU4) provided herein, the combination therapy is for the treatment of mBC.

Further provided herein is the use (GM1) of a combination therapy described herein comprising GDC-9545 or a pharmaceutically acceptable salt thereof and GDC-0077 for the manufacture of a medicament for the treatment of laBC or mBC as described herein. Still further provided herein is the use (GM2) of a combination therapy described herein comprising GDC-9545 or a pharmaceutically acceptable salt thereof and GDC-0077 for the manufacture of a medicament for the treatment of mBC as described herein. Further provided herein is the use (GM3) of a combination therapy described herein comprising GDC-9545 or a pharmaceutically acceptable salt thereof and GDC-0077 for the manufacture of a medicament for the treatment of laBC as described herein.

Further provided herein is the use (GM4) of a combination therapy described herein comprising GDC-9545 or a pharmaceutically acceptable salt thereof and GDC-0077 for the manufacture of a medicament for the treatment of laBC or mBC as described herein comprising a dosing regimen comprising: (i) administering GDC-9545 or a pharmaceutically acceptable salt thereof QD on days 1-28 of a first 28-day cycle; and (ii) administering GDC-0077 QD on days 1-28 of the first 28-day cycle. In one embodiment of the use (IM4) provided herein, the combination therapy is for the treatment of laBC. In another embodiment of the use (IM4) provided herein, the combination therapy is for the treatment of mBC.

Further provided herein is the use (GM5) of a combination therapy described herein comprising GDC-9545 or a pharmaceutically acceptable salt thereof and GDC-0077 for the manufacture of a medicament for the treatment of laBC or mBC as described herein comprising a dosing regimen comprising: (i) administering 30 mg GDC-9545 or a pharmaceutically acceptable salt thereof QD on days 1-28 of a first 28-day cycle; and (ii) administering 9 mg GDC-0077 on days 1-28 of the first 28-day cycle. In one such embodiment, the dosing regimen includes 2 or more cycles as described herein. In one embodiment of the use (GM5) provided herein, the combination therapy is for the treatment of laBC. In another embodiment of the use (GM5) provided herein, the combination therapy is for the treatment of mBC.

Also provided herein are methods of inhibiting tumor growth or producing tumor regression in a patient described herein by administering a combination therapy described herein. In one embodiment provided herein is a method of inhibiting tumor growth in a patient having laBC described herein by administering a combination therapy comprising administering GDC-9545 or a pharmaceutically acceptable salt thereof and GDC-0077 in one or more 28-day cycles as described herein. In one embodiment provided herein is a method of inhibiting tumor growth in a patient having mBC described herein by administering a combination therapy comprising administering GDC-9545 or a pharmaceutically acceptable salt thereof and GDC-0077 in one or more 28-day cycles as described herein.

In one embodiment provided herein is a method of producing or improving tumor regression in a patient having mBC described herein by administering a combination therapy comprising administering GDC-9545 or a pharmaceutically acceptable salt thereof and GDC-0077 in one or more 28-day cycles as described herein. In one embodiment provided herein is a method of producing or improving tumor regression in a patient having laBC described herein by administering a combination therapy comprising administering GDC-9545 or a pharmaceutically acceptable salt thereof and GDC-0077 in one or more 28-day cycles as described herein.

The development of combination treatments poses challenges including, for example, the selection of agents for combination therapy that may lead to improved efficacy while maintaining acceptable toxicity. One particular challenge is the need to distinguish the incremental toxicity of the combination. In one embodiment of the methods described herein the combination therapy described herein (e.g. GDC-9545 or a pharmaceutically acceptable salt thereof and GDC-0077) is administered in a dosing regimen comprising a staggered dosing schedule. In one such embodiment, the patient has a reduced number or grade of adverse events (AEs) comparable to a control (e.g. SOC therapy, treatment with one agent described herein (e.g. GDC-9545 or GDC-0077) alone).

In one embodiment of the methods described herein, the dosing regimen reduces the number or frequency of grade 2 or grade 3 or higher grade adverse event comparable to administration of either GDC-9545 or GDC-0077 alone. In one such embodiment, the dosing regimen eliminates the number or frequency of grade 3 or higher AEs. In one embodiment, the dosing regimen reduces the grade of bradycardia. In another embodiment, the dosing regimen reduces the grade of hyperglycemia. In another embodiment, the dosing regiment reduces or eliminates ocular toxicities such as lens fiber swelling, separation of lens fibers and/or accumulation of subscapsualr proteinaceous material.

In another embodiment of the methods described herein the dosing reduces the number or frequency of grade 2 or grade 3 or higher grade adverse event comparable to administration of either agent alone.

It is generally understood that the when an adverse event occurs, four options exist: (1) continue treatment as-is with optional concomitant therapy; (2) adjust the dose of one or more agents in the dosing regiment; (3) suspend administration of one or more agents in the dosing regimen; or (4) discontinue administration of one or more agents in the dosing regimen. In one embodiment, GDC-9545 is not adjusted.

In one embodiment, the adverse event(s) experienced by a patient described herein undergoing treatment with a combination therapy described herein are comparably reduced as described herein.

In some embodiments, where a patient experiences one or more AEs selected from the group consisting of hyperglycemia, ocular toxicities (e.g. as described herein), and bradycardia from treatment with a combination therapy described herein, the severity is Grade 2 or less. In one embodiment, a patient described herein does not experience one or more AEs selected from the group consisting of hyperglycemia, ocular toxicities (e.g. as described herein), and bradycardia from treatment with a combination therapy described herein, where the severity of the AE is higher than Grade 2.

### Biomarkers

Breast cancer is a heterogeneous disease with many distinct subtypes as defined by molecular signatures and a diverse array of mutational profiles. Patients described herein can be tested for ER+ HER2- laBC or mBC using diagnostic methods, or kits to inform treating or predict of responsiveness of a pateint to the combination therapies described herein. In one embodiment, a patient can be tested by determining an ER pathway activity score such as those described in US Patent Application Publication 20200082944. In some embodiments, a patient sample is taken and tested to determine an ER pathway activity score. The score can be calculated using a 41-gene signature by subtracting an E2-repressed score (as determined from the average z-scored expression of genes comprising BAMBI, BCAS1, CCNG2, DDIT4, EGLN3, FAM171B, GRM4, IL1R1, LIPH, NBEA, PNPLA7, PSCA, SEMA3E, SSPO, STON1, TGFB3, TP53INP1, and TP53INP2) from an E2-induced score (as determined from the average z-scored expression of genes set forth in AGR3, AMZ1, AREG, C5AR2, CELSR2, CT62, FKBP4, FMN1, GREB1, IGFBP4, NOS1AP, NXPH3, OLFM1, PGR, PPM1J, RAPGEFL1, RBM24, RERG, RET, SGK3, SLC9A3R1, TFF1, and ZNF703).

In one embodiment, the sample from the patient used for determining the ER pathway activity score is a tumor tissue sample, (e.g., a formalin-fixed paraffin-embedded (FFPE), a fresh frozen (FF), an archival, a fresh, or a frozen tumor tissue sample).

In some instances, a patient described herein is administered a combination therapy described herein where the measured ER pathway activity score is be between about -1.0 to about -0.2 (e.g., between about -0.9 to about -0.2, e.g., between about -0.8 to about -0.2, e.g., between about -0.7 to about -0.2, e.g., between about -0.6 to about - 0.2, e.g., between about -0.5 to about -0.2, e.g., between about -0.4 to about -0.2, or e.g., between about -0.3 to about -0.2). In some instances, the ER activity score from the sample may be less than -1.0.

In some embodiments, samples of patients described herein can be assessed for additional biomarkers in an effort to identify factors that may correlate with the safety and efficacy of the study treatments.

In one embodiment of the methods described herein, NGS, whole genome sequencing (WGS), other methods, or a combination thereof can be used for DNA obtained from blood samples and tumor tissue from patients described herein. Such samples may be analyzed to identify germline and somatic alterations that are predictive of response to study drug, are associated with progression to a more severe disease state, are associated with acquired resistance to study drug, or can increase the knowledge and understanding of disease biology. In one embodiment, a patient sample is evaluated for the level of expression of Ki67 using one ore more techniques as described above.

In one embodiment, a patient sample is evaluated or tested for one or more PIK3CA mutations corresponding to positions 88, 106, 111, 118, 345, 420, 453, 542, 545, 546, 1043, 1047 and 1049. In one embodiment, a patient sample is evaluated or tested for one or more PIK3CA mutations corresponding to one or more of positions H1047, E545, E542, Q546, N345, C420, M1043, G1049, E453, K111, G106, G118, and R88. In one embodiment, a patient sample is evaluated or tested for one or more PIK3CA mutations corresponding to one or more positions selected from the group consisting of H1047D/I/L/N/P/Q/R/T/Y, E545A/D/G/KIL/Q/R/V, E542A/D/G/K/Q/R/V, Q546E/H/K/L/P/R, N345D/H/I/K/S/T/Y, C420R, M1043I/T/V, G1049A/C/D/R/S, E453A/D/G/K/Q/V, K111N/R/E, G106A/D/R/S/V, G118D, and R88Q. In one embodiment, a patient sample is evaluated or tested for one or more PIK3CA mutations corresponding to one or more positions selected from the group consisting of E542K, E545K, Q546R, H1047L and H1047R. In one embodiment, a patient sample is evaluated or tested for one or more PIK3CA mutations corresponding to one mutation selected from the group consisting of E542K, E545K, Q546R, H1047L and H1047R, and a second mutation selected from the group consisting of E453Q/K, E726K and M1043L/I. In one embodiment, a patient sample is evaluated or tested for one or more PIK3CA mutations corresponding to positions selected from the group consisting of E542K + E453Q/K, E542K + E726K, E542K + M1043L/I; E545K + E453Q/K, E545K + E726K, E545K + M1043L/I; H1047R + E453Q/K, and H1047R + E726K.

In one embodiment, the patient has breast cancer expressing a PIK3CA mutant comprising a mutation corresponding to positions selected from the group consisting of H1047D/I/L/N/P/Q/R/T/Y, E545A/D/G/KIL/Q/R/V, E542A/D/G/K/Q/R/V, Q546E/H/K/L/P/R, N345D/H/I/K/S/T/Y, C420R, M1043I/T/V, G1049A/C/D/R/S, E453A/D/G/K/Q/V, K111N/R/E, G106A/D/R/S/V, G118D, and R88Q. In one embodiment, the patient has breast cancer expressing a PIK3CA mutant comprising a mutation corresponding to positions selected from the group consisting of E542K, E545K, Q546R, H1047L and H1047R. In one embodiment, the patient has mutant PIK3CA comprising a mutation corresponding to positions containing one mutation selected from the group consisting of E542K, E545K, Q546R, H1047L and H1047R, and a second mutation selected from the group consisting of E453Q/K, E726K and M1043L/I. In one embodiment, the patient has breast cancer expressing a PIK3CA mutant comprising a mutation corresponding to positions selected from the group consisting of E542K + E453Q/K, E542K + E726K, E542K + M1043L/I; E545K + E453Q/K, E545K + E726K, E545K + M1043L/I; H1047R + E453Q/K, and H1047R + E726K. In one embodiment, PIK3CA-mutant tumor status is assessed by either central testing of blood or local testing of blood or tumor tissue. In one embodiment, the central test for identification of eligible *PIK3CA* mutations is the FoundationOne Liquid Clinical Trial Assay performed at Foundation Medicine, Inc. In one embodiment, the local tests of blood or tumor tissue is performed using PCR- or a NGS-based assay at a CLIA-certified or equivalent laboratory.

### Embodiments:

Provided below are exemplary embodiments of the invention.

Embodiment No 1. A combination therapy comprising GDC-9545 or a pharmaceutically acceptable salt thereof administered QD on days 1-28 of a first 28-day cycle and GDC-0077 administered QD on days 1-28 of the first 28-day cycle.

Embodiment No 2. The combination therapy of embodiment 1, wherein GDC-0077 is administered at a dose of 9 mg.

Embodiment No 3. The combination therapy of embodiment 1 or embodiment 2, wherein GDC-9545 or a pharmaceutically acceptable salt thereof is administered at an amount of 30 mg.

Embodiment No 4. The combination therapy of any one of embodiments 1-3, wherein the dosing regimen comprises 2, 3, 4, 5, 6, 7, 8, 9, 10, 11, 12, 13, 14, 15, 16, 17, 18, 19, 20, 21, 22, 23, 24, 30, 36, 42, 48, 54, 60, 66, or 72 cycles.

Embodiment No 5. The combination therapy of any one of embodiments 1-4, wherein the dosing regimen comprises about 2-72, 2-66, 2-60, 2-54, 2-48, 2-42, 2-36, 2-30, 2-24, 2-18, or 2-12 cycles.

Embodiment No 6. A method of treating estrogen receptor-positive and HER2-negative locally advanced breast cancer (laBC) or metastatic breast cancer (mBC) in a patient having estrogen receptor-positive and HER2-negative laBC or mBC, the method comprising administering to the patient a combination therapy comprising GDC-9545 or a pharmaceutically acceptable salt thereof and GDC-0077, wherein said combination therapy is administered over one or more 28-day cycles.

Embodiment No 7. A method of treating estrogen receptor-positive and HER2-negative locally advanced breast cancer (laBC) or metastatic breast cancer (mBC) in a patient having receptor-positive and HER2-negative laBC or mBC, the method comprising administering to the patient a combination therapy comprising a dosing regimen comprising:
i. administering GDC-9545 or a pharmaceutically acceptable salt thereof QD on days 1-28 of a first 28-day cycle; and
ii. administering GDC-0077 QD on days 1-28 of the first 28-day cycle.

Embodiment No 8. The method of embodiment 6 or embodiment 7, wherein GDC-0077 is administered at a dose of 9 mg.

Embodiment No 9. The method of any one of embodiments 6-8, wherein GDC-9545 or a pharmaceutically acceptable salt thereof is administered at an amount of 30 mg.

Embodiment No 10. The method of any one of embodiments 7-9, wherein the dosing regimen comprises 2, 3, 4, 5, 6, 7, 8, 9, 10, 11, 12, 13, 14, 15, 16, 17, 18, 19, 20, 21, 22, 23, 24, 30, 36, 42, 48, 54, 60, 66, or 72 cycles.

Embodiment No 11. The method of any one of embodiments 7-9, wherein the dosing regimen comprises about 2-72, 2-66, 2-60, 2-54, 2-48, 2-42, 2-36, 2-30, 2-24, 2-18, or 2-12 cycles.

Embodiment No 12. The method of any one of embodiments 6-11, wherein the patient is premenopausal.

Embodiment No 13. The method of any one of embodiments 6-12, wherein the patient is tested for the presence of a mutation of one or more of estrogen receptor, prostaglandin receptor, or Ki67.

Embodiment No 14. The method of any one of embodiments 6-13, wherein the patient has a tumor comprising one or more mutation of PIK3CA at positions corresponding to R88Q; G106, K111, G118, N345, C420, E453, E542, E545, Q546, M1043, H1047, or G1049, or a combination thereof.

Embodiment No 15. The method of embodiment 14, wherein the mutation of PIK3CA corresponds to
R88Q;
G106A/D/R/S/V;
K111N/R/E;
G118D;
N345D/H/I/K/S/T/Y;
C420R;
E453A/D/G/K/Q/V;
E542A/D/G/K/Q/R/V;
E545A/D/G/K/L/Q/R/V;
Q546E/H/K/L/P/R;
M1043I/T/V;
H1047D/I/L/N/P/Q/R/T/Y; or
G1049A/C/D/R/S;
or a combination thereof.

Embodiment No 16. The method of embodiment 14 or embodiment 15, wherein the mutation of PIK3CA comprises one mutation selected from the group consisting of E542K, E545K, Q546R, H1047L and H1047R, and a second mutation selected from the group consisting of E453Q/K, E726K and M1043L/I.

Embodiment No 17. The method of embodiment 16, wherein the mutation of PIK3CA is selected from a double mutation selected from the group consisting of E542K + E453Q/K, E542K + E726K, E542K + M1043L/I; E545K + E453Q/K, E545K + E726K, E545K + M1043L/I; H1047R + E453Q/K, and H1047R + E726K.

Embodiment No 18. The method of any one of embodiments 6-17, wherein the patient has reduced adverse events (AEs) comparable to a control.

Embodiment No 19. The method of embodiment 18, wherein the patient has reduced severity of one or more AEs selected from the group consisting of hepatotoxicity, bradycardia, hyperglycemia, stomatitis/oral mucositis, rash, diarrhea/colitis, or pneumonitis, or a combination thereof comparable to a control.

Embodiment No 20. The method of embodiment 18, wherein the patient has the same level or reduced level of bradycardia or hyperglycemia following administration of the combination therapy compared to the control.

Embodiment No 21. The method of any one of embodiments 6-20, wherein the patient has an increased overall survival (OS) comparable to a control.

Embodiment No 22. The method of embodiment 21, wherein the patient has an increase of 1, 2, 3, 4, 5, 6, 7, 8, 9, 10, 11, 12, 14, 16, 18, 20, 24 or more months comparable to a control.

Embodiment No 23. The method of any one of embodiments 6-22, wherein duration of response to the combination therapy is increased compared to a control.

Embodiment No 24. The method of embodiment 23, wherein the duration of response is increased by at least 1-3, 2-6, 3-8, 4-10, 5-12, 6-15, 8-20, or 1-24 months.

Embodiment No 25. The method of any one of embodiments 6-24, wherein a patient has increased clinical benefit rate compared to a control.

Embodiment No 26. The method of any one of embodiments 6-25, wherein a patient has increased progression-free survival compared to a control.

Embodiment No 27. The method of embodiment 26, wherein the increase is at least 2, 4, 6, 8, 10, 12, 14, 16, 18, 20, 22, 24, 26, 28, 30, 36, 42, 48, 50, 54, 60, 66, or 72 months.

Embodiment No 28. The method any one of embodiments 18-27, wherein the control is GDC-9545 administered alone or palbociclib administered in combination with letrozole.

Embodiment No 29. The method of any one of embodiments 6-28, wherein the patient has not received prior chemotherapy before administration of the combination therapy.

Embodiment No 30. The method of any one of embodiments 6-28, wherein the patient has not been previously treated with a PI3K, AKT, or mTOR inhibitor.

Embodiment No 31. The method of any one of embodiments 6-28, wherein the patient has been previously treated with fulvestrant.

Embodiment No 32. Use of a combination therapy comprising GDC-9545 or a pharmaceutically acceptable salt thereof and GDC-0077 for the treatment of laBC or mBC.

Embodiment No 33. Use of a combination therapy comprising GDC-9545 or a pharmaceutically acceptable salt thereof and GDC-0077 for the manufacture of a medicament for the treatment of laBC or mBC.

Embodiment No 34. The use of embodiment 33, wherein the combination therapy comprises a dosing regimen comprising: (i) administering 30 mg GDC-9545 or a pharmaceutically acceptable salt thereof QD on days 1-28 of a first 28-day cycle; and (ii) administering mg GDC-0077 QD on days 1-28 of the first 28-day cycle.

Embodiment No 35. The use of any one of embodiments 32-34, wherein the combination therapy is for the treatment of laBC.

Embodiment No 36. The use of any one of embodiments 32-34, wherein the combination therapy is for the treatment of mBC.

Embodiment No 37. A method of inhibiting tumor growth in a patient having laBC or mBC, the method comprising administering a combination therapy comprising GDC-9545 or a pharmaceutically acceptable salt thereof and GDC-0077 in one or more 28-day cycles.

Embodiment No 38. A method of producing or improving tumor regression in a patient having laBC or mBC, the method comprising administering a combination therapy comprising GDC-9545 or a pharmaceutically acceptable salt thereof and GDC-0077 in one or more 28-day cycles.

The following Examples are presented by way of illustration, not limitation.

### Examples:

### Example 1

The role of estrogen in breast cancer etiology and disease progression is well established (Colditz et al. N Engl J Med 1995;332:1589-93). Modulation of estrogen activity and/or synthesis is one therapeutic approach in patients with ER+ breast cancer.

Despite the effectiveness of available therapies for patients with ER+, locally advanced or metastatic disease including endocrine therapy (ET) and combinations of endocrine and targeted therapy, many patients ultimately relapse or develop resistance to these agents and therefore require further treatment for optimal disease control. However, growth and survival of the majority of tumors are thought to remain dependent on ER signaling, despite becoming refractory to Als or tamoxifen. Patients with ER+ breast cancer can still respond to second- or third-line ET after progression on prior therapy (Di Leo et al. J Clin Oncol. 2010;28:4594-600; Baselga et al. N Engl J Med. 2012;366:520-9). Without being bound by any particular theory, there is evidence that in the endocrine-resistant state, the ER can signal in a ligand-independent manner (Miller et al. J Clin Invest 2010;120:2406-13; Van Tine et al. Cancer Discov 2011;1:287-8). An agent (or combination of agents) capable of targeting both ligand-dependent and ligand-independent ER signaling has the potential to improve treatment outcomes in patients with ER+ breast cancer.

*ESR1* mutations appear to be a major mechanism of acquired resistance to Als and are associated with poorer outcomes (Schiavon et al. Sci Transl Med 2015;7:313ra182; Chandarlapaty et al. JAMA Oncol 2016;2:1310-15; Fribbens et al. J Clin Oncol 2016;34:2961-8). The prevalence of *ESR1* mutation appears to range from about 25%-40% after Al exposure but only in 2%-3% of ET-naive patients (Chandarlapaty et al. 2016). This illustrates that *ESR1* becomes one important oncogenic driver under Al-selection pressure. Studies have identified mutations in *ESR1* encoding ER-α (primarily Y537S and D538G) affecting the ligand binding domain "LBD" of the ER-α (Segal and Dowsett Clin Cancer Res 2014;20:1724-6). Studies using clinical samples and nonclinical models describe ER antagonists appear efficacious against ligand-independent, constitutively active ER-mutated receptors and may have therapeutic benefit for patients that were resistant to Als (Li et al. Cell Rep. 2013;4:1116-30; Merenbakh-Lamin et al. Cancer Res 2013;73:6856-64; Robinson et al. Nat Genet 2013;45:1466-51; Toy et al. Nat Genet 2013;45:1439-45; Alluri et al. Breast Cancer Res 2014;16:494; Segal and Dowsett Clin Cancer Res 2014;20:1724-6; Jeselsohn et al. Nat Rev Clin Oncol 2015; 12:573-83; Niu et al. Onco Targets Ther. 2015;8:3323-8; Schiavon et al. Sci Transl Med 2015;7:313ra182; Chu et al. Clin Cancer Res 2016; 22:993-9).

Selective estrogen receptor degraders (SERDs) can block endocrine-dependent and endocrine-independent ER signaling and have been recognized to offer a therapeutic approach to ER+ metastatic breast cancer. Fulvestrant, a first-generation SERD, binds, blocks, and degrades the ER, leading to inhibition of estrogen signaling through the ER. Fulvestrant has also shown benefit over anastrozole in frontline patients, as demonstrated in one study (NCT01602380). However, bioavailability and delivery of fulvestrant hinder its effectiveness adminstration.

Nonclinical studies comparing drug exposure and in vitro potency of GDC-9545 versus fulvestrant demonstrated that human steady-state total drug exposure of GDC-9545 at 30 mg once a day (QD) is approximately 10-fold higher than the steady-state exposure of fulvestrant 500 mg intramuscular (IM) monthly. Furthermore, the lower plasma protein binding of GDC-9545 provides higher free concentration of GDC-9545 than fulvestrant. In in vitro cell and biochemical assays, GDC-9545 exhibited up to 10-times higher potency than fulvestrant both in wild-type and ESR1-mutant contexts. Fulvestrant, when dosed according to a clinically relevant dosing scheme, was less efficacious than GDC-9545 in the assessed xenograft models.

PI3K, Akt, and mammalian target of rapamycin (mTOR) are major nodes in the PI3K/Akt/mTOR intracellular signaling pathway and are critical for cell-cycle modulation, cell growth, metabolism, motility, and survival (Cantrell, J Cell Sci 2001; 114:1439-45; Hanahan and Weinberg, Cell, 2011;144:646-74; Vanhaesebroeck et al. Nat Rev Mol Cell Biol 2012;13:195-203). The PI3K/Akt/mTOR pathway is generally activated following ligand-receptor tyrosine kinase interactions. Under physiologic conditions, the main role of PI3K is to facilitate the metabolism of inositol phospholipids for intracellular signal transduction.

There are three classes of PI3K, with Class I considered as the most responsive to external stimuli. Class I PI3Ks are composed of two subunits: a p110 catalytic subunit and a regulatory adapter subunit, p85. There are four isoforms of the p110 catalytic subunit of PI3K: α, β, γ, and δ. These four isoforms are the respective products of the genes *PIK3CA, PIK3CB, PIK3CG,* and *PIK3CD. PIK3CA* and *PIK3CB* are expressed in all cells, while *PIK3CD* is primarily expressed in leukocytes and *PIK3CG* is expressed in multiple tissues, including pancreas, skeletal muscle, liver, and heart.

Dysregulation of the PI3K/Akt/mTOR signaling pathway has been described in multiple solid tumor malignancies including, for example, glioblastoma, colorectal, gastric, lung, endometrial, ovarian, prostate, and breast cancers (Gustin et al. Curr Cancer Drug Targets 2008;8:733-40). Pathway activation may occur through multiple mechanisms such as loss of the tumor suppressor phosphatase and tensin homolog *(PTEN*), amplification or somatic mutations in PIK3CA, mutations in AKT, mutations in the regulatory subunit p85, mutations and/or amplification of upstream-receptor tyrosine kinases, mutations in *RAS,* and loss of liver kinase B1 (LKB1), type II inositol polyphosphate-4-phosphatase (INPP4B), or tuberous sclerosis (Staal Proc Natl Acad Sci USA 1987;84:5034-7; Cheng et al. Proc Natl Acad Sci USA 1992;89:9267-71; Bellacosa et al. Int J Cancer 1995;64:280-5; Li et al. Science 1997;275:1943-7; Steck et al. Nat Genet 1997;15:356-62; Aoki et al. Proc Natl Acad Sci USA 1998;95:14950-5). Activating mutations in the *PIK3CA* gene are the most common genomic alterations and occur primarily in exons 9 and 20, which encode the helical and kinase domains of the PI3K-α protein (Bachman et al. Cancer Biol Ther 2004;3:772-5; Samuels et al. Science 2004;304:554).

Up to 70% of breast cancers can have some form of molecular aberration of the PI3K/Akt/mTOR pathway (Cancer Genome Atlas Network Nature 2012;490:61-70). Hyperactivation of the PI3K/Akt/mTOR signaling pathway was proven to promote both de novo and acquired resistance to hormone therapy in ER+ breast cancer cell lines and xenograft models (Sabnis et al. Clin Cancer Res 2007;13:2751-7). Furthermore, simultaneous blocking of the PI3K/Akt/mTOR pathway with everolimus and the ER pathway with letrozole results in greater anti-tumor activity than either agent alone (Boulay et al. Clin Cancer Res 2005;11:5319-28). In addition, evidence of baseline PI3K activation was found to be predictive of a poor prognosis after adjuvant ET (Miller et al. J Clin Invest 2010;120:2406-13).

These data provide support for the hypothesis that blocking PISK/Akt/mTOR pathway signaling may have a therapeutic benefit in patients with ER+, HER2-negative breast cancer.

GDC-0077 is a potent and selective inhibitor of the Class I alpha isoform of phosphatidylinositol 3-kinase (PI3K-α), which contains the p100α catalytic subunit (encoded by the *PI3KCA* gene). GDC-0077 provides >300-fold less potent biochemical inhibition of other Class I PI3Ks, including the β, δ, and γ isoforms, and shows increased potency in tumor cells bearing mutant p110α over cells bearing wild-type p110α. Without being bound by any particular theory, GDC-0077 appears to exert its activity by binding to the adenosine 5'-triphosphate binding site of p110α, thereby inhibiting the phosphorylation of membrane-bound phosphatidylinositol 4,5-bisphosphate (PIP₂) to phosphatidylinositol 3,4,5-trisphophate (PIP₃). This inhibition decreases downstream activation of pathway effectors including Akt and S6RP, resulting in decreased cellular proliferation, metabolism, and angiogenesis. Nonclinical studies have demonstrated that GDC-0077 specifically degrades mutant p110α, inhibits proliferation, and induces apoptosis in PIK3CA-mutant breast cancer cell lines, inhibits tumor growth in human breast cancer xenograft models harboring PIK3CA mutations, and reduces downstream PI3K pathway markers, including pAkt, PRAS40 phosphorylated at threonine 246 (pPRAS40), and S6RP phosphorylated at serine 235/236 (pS6RP).

In one study using single agent administration of GDC-0077, among 20 patients with measurable disease treated with a 6-mg, 9-mg, or 12-mg QD starting dose of GDC-0077 as a single agent, 5 patients (25%) achieved PRs, while 9 patients (45%) achieved clinical benefit (defined as the percentage of patients achieving confirmed Response Evaluation Criteria in Solid Tumors, Version 1.1 [RECIST v1.1]-defined CR, PR, and/or SD [non-complete response/non-progressive disease for patients with non-measurable disease at baseline] for ≥ 24 weeks). Further, 14 of the 20 patients were enrolled at the 6-mg or 9-mg QD starting dose, and 3 patients had received prior treatment with a CDK4/6i.

GDC-9545 is a potent, orally bioavailable ER-α antagonist and inducer of ER-α degradation that competes with estrogens for binding to the ER with low nanomolar potency; it is being developed for the treatment of patients with ER+ advanced or metastatic breast cancer. GDC-9545 has demonstrated robust nonclinical activity in ER+ breast cancer models of ESR1-wild type and ESR1-mutation-bearing disease. Furthermore, fulvestrant, an approved SERD molecule, when dosed according to a clinically relevant dosing scheme, was less efficacious than GDC-9545 in the assessed xenograft models).

Patients will receive GDC-9545 as an immediate release capsule at a dose of 30 mg PO QD during each 28 day cycle and GDC-0077 as an immediate release tablet at a dose of 9 mg PO QD on Days 1-28 of each 28 day cycle.

Patients administered GDC-9545 should be taken PO at approximately the same time each day starting with Day 1 of Cycle 1, and on Day 1 of each 28-day cycle thereafter. If a dose is not taken within 6 hours after the scheduled dosing, it will be considered missed. If a dose is missed or vomited, the patient should resume dosing with the next scheduled dose; missed or vomited doses will not be made up. GDC-0077 should be taken at approximately the same time each day, and no later than 9 hours after the scheduled time.

Patients administered GDC-9545 and GDC-0077 are permitted to use the following concomitant therapies:
a. Symptomatic anti-emetics, anti-diarrheal therapy, and other palliative and supportive care for disease-related symptoms.
b. Pain medications administered per standard clinical practice.
c. Bone-sparing agents (e.g., bisphosphonates, denosumab) for the treatment of osteoporosis/osteopenia or for palliation of bone metastases, provided patient was on stable doses prior to Day 1 of Cycle 1.

Patients administered GDC-9545 and GDC-0077 are not permitted to use the following concomitant therapies:
a. Investigational therapy (other than protocol-mandated study treatment) is within 28 days prior to first dose of GDC9545 and GDC-0077.
b. Any concomitant therapy intended for the treatment of cancer including, but not limited to, chemotherapy, immunotherapy, biologic therapy, radiotherapy, or herbal therapy is prohibited.
c. Hormone replacement therapy, topical estrogens (including any intra-vaginal preparations), megestrol acetate, and selective ER modulators (e.g., raloxifene).
d. Primary prophylactic use of hematopoietic growth factors (e.g., erythropoietins, granulocyte colony-stimulating factor, and granulocyte-macrophage colony-stimulating factor).
e. Radiotherapy for unequivocal progressive disease, with the exception of new brain metastases in the setting of systemic response as follows:
   Patients who have demonstrated control of their systemic disease (defined as having received clinical benefit [i.e., a PR, CR, or SD for ≥ 24 weeks]), but who have developed isolated brain metastases treatable with radiation.
   ET (i.e., GDC-9545) may be administered concomitantly with radiotherapy.
f. Quinidine or other anti-arrhythmic agents

GDC-9545 may temporarily be suspended in patients experiencing toxicity considered to be related to study treatment. GDC-0077 may temporarily be suspended in patients experiencing toxicity considered to be related to study treatment. If either GDC-9545 or GDC-0077 is discontinued, the other drug can be continued if the patient is likely to derive clinical benefit, as determined by the investigator.

### Example 2

Combination of GDC-9545 and GDC-007 was investigated and compared to efficacy of each single agent and to an Al+palbociclib control. ESR1 wildtype MCF-7 (PIK3CA.E545K) and T-47D (PIK3CA.H1047R) cells, and engineered ESR1 mutant cells [MCF-7.ER.Y537S, T-47D.ER.D538G] were grown under standard cell culture conditions in RPMI media with 10% fetal bovine serum. One day prior to the addition of drugs, cells were seeded in 384-well plates in phenol red-free RPMI with 10% charcoal-stripped fetal bovine serum in order to deprive the cells of hormones. Single agent 300 nM GDC-9545, 80 nM GDC-0077, 125 nM palbociclib or a combination thereof, were tested in quadruplicate in the presence of 0.1 nmol/L estradiol (E2) during a 6-day or 8-day incubation period. The absence of E2 in the media (- E2) serves to mimic treatment with an Aromatase Inhibitor (Al). The combination of E2 withdrawal plus palbociclib - reflecting the current standard of care for metastatic breast cancer - was included. Drug concentrations of GDC-0077 and palbociclib were chosen to be clinically relevant, based on human exposures. 300 nM GDC-9545 is a saturating concentration that is lower than the human dose equivalent of 30 mg. The relative number of viable cells at the 6/8-day timepoint was determined by CyQUANT (ThermoFisher, C7026).

In MCF-7 cells expressing wildtype ESR1, E2 withdrawal resulted in a modest reduction of cell proliferation over the incubation period, which is much enhanced by the addition of palbociclib (FIG. 1). Direct ER antagonism by GDC-9545 had a greater impact on cell viability versus estrogen withdrawal. Combining the PI3K (p110α-selective) inhibitor, GDC-0077, with GDC-9545 further enhanced the anti-proliferative effect of GDC-9545. Cells treated with a combination of GDC-9545 and GDC-0077 displayed significantly reduced viability versus cells subject to estrogen withdrawal plus CDK4/6 inhibition and to administration of each agent alone (FIG. 1).

In MCF-7 cells engineered to express an ESR1 mutation (ER.Y537S), E2 withdrawal alone failed to inhibit cell proliferation, consistent with the estrogen-independent ER activity previously established for this ER mutant (FIG. 2). Combining palbociclib with E2 withdrawal likewise had little anti-proliferative effect. In contrast, direct ER antagonism by GDC-9545 reduced cell numbers relative to the control condition. When combined with GDC-0077, the combination demonstrated significantly more anti-proliferative effect than either agent alone and E2 withdrawal+CDK/4/6 inhibition (FIG. 2).

In T-47D cells that are wildtype for ESR1, the suppression of ER activity through either E2 withdrawal or GDC-9545 treatment resulted in reduced cell proliferation relative to control treated cells (FIG. 3). The addition of CDK4/6 inhibition to E2 withdrawal resulted in a greater impact to cell viability that E2 withdrawal alone, but does not achieve the same anti-proliferative activity as the combination of GDC-9545 and GDC-0077 (FIG. 3). Likewise, addition of GDC-0077 alone or GDC-9545 alone did not achieve the same anti-proliferative activity as the combination of the agents.

T-47D cells engineered to express an ESR1 mutation (ER.D538G) are only modestly impacted by E2 withdrawal. Co-treatment with palbociclib improved the anti-proliferative effect (FIG. 4). Single agent GDC-9545 was considerably more impactful than E2 withdrawal in this cell line, and the anti-proliferative effect of GDC-9545 plus GDC-0077 was greater than that of combining palbociclib with E2 withdrawal (Figure 1D). Likewise, the combination of GDC-9545 and GDC-0077 showed considerable anti-proliferative effect comparable to each agent alone.

The anti-proliferative effect of the combination of GDC-9545 and GDC-0077 in PIK3CA mutant cell lines, harboring either mutant or wildtype ESR1, is greater than that of GDC-9545 alone, GDC-0077 alone, and also greater than that of combining palbociclib with E2 withdrawal. These preclinical data demonstrate the enhanced anti-proliferative effect of the GDC-9545+GDC-0077 combination over all other tested agents.

Throughout this specification and the claims, the words "comprise," "comprises," and "comprising" are used in a non-exclusive sense, except where the context requires otherwise. It is understood that embodiments described herein include "consisting of" and/or "consisting essentially of" embodiments.

Where a range of values is provided, it is understood that each intervening value, to the tenth of the unit of the lower limit, unless the context clearly dictates otherwise, between the upper and lower limit of the range and any other stated or intervening value in that stated range, is encompassed herein. The upper and lower limits of these small ranges which can independently be included in the smaller rangers is also encompassed herein, subject to any specifically excluded limit in the stated range. Where the stated range includes one or both of the limits, ranges excluding either or both of those included limits are also included herein.

Many modifications and other embodiments of the inventions set forth herein will come to mind to one skilled in the art to which these inventions pertain having the benefit of the teachings presented in the foregoing descriptions and the associated drawings. Therefore, it is to be understood that the inventions are not to be limited to the specific embodiments disclosed and that modifications and other embodiments are intended to be included within the scope of the appended claims. Although specific terms are employed herein, they are used in a generic and descriptive sense only and not for purposes of limitation.

## Claims

1. A combination therapy comprising GDC-9545 or a pharmaceutically acceptable salt thereof and GDC-0077 or a pharmaceutically acceptable salt thereof.

2. The combination therapy according to claim 1, wherein said GDC-9545 or a pharmaceutically acceptable salt thereof is administered QD on days 1-28 of a first 28-day cycle and GDC-0077 or a pharmaceutically acceptable salt thereof is administered QD on days 1-28 of the first 28-day cycle.

3. The combination therapy of claim 2, wherein GDC-0077 is administered at a dose of 9 mg and GDC-9545 or a pharmaceutically acceptable salt thereof is administered at an amount of 30 mg.

4. The combination therapy of claim 1 for use in a method of treating estrogen receptor-positive and HER2-negative locally advanced breast cancer (laBC) or metastatic breast cancer (mBC) in a patient having receptor-positive and HER2-negative laBC or mBC, the method comprising administering to the patient a combination therapy comprising a dosing regimen comprising:
i. administering GDC-9545 or a pharmaceutically acceptable salt thereof QD on days 1-28 of a first 28-day cycle; and
ii. administering GDC-0077 QD on days 1-28 of the first 28-day cycle.

5. The combination therapy for use of claim 4, wherein GDC-0077 is administered at a dose of 9 mg.

6. The combination therapy for use of claim 4 or 5, wherein GDC-9545 or a pharmaceutically acceptable salt thereof is administered at an amount of 30 mg.

7. The combination therapy for use of any one of claims 4-6, wherein the patient is premenopausal.

8. The combination therapy for use of any one of claims 4-7, wherein the patient is tested for the presence of a mutation of one or more of estrogen receptor, prostaglandin receptor, or Ki67.

9. The combination therapy for use of any one of claims 4-8, wherein the patient has a tumor comprising one or more mutation of PIK3CA at positions corresponding to R88Q; G106, K111, G118, N345, C420, E453, E542, E545, Q546, M1043, H1047, or G1049, or a combination thereof.

10. The combination therapy for use of claim 9, wherein the mutation of PIK3CA corresponds to
R88Q;
G106A/D/R/SN;
K111N/R/E;
G118D;
N345D/H/I/K/S/T/Y;
C420R;
E453A/D/G/K/QN;
E542A/D/G/K/Q/RN;
E545A/D/G/K/L/Q/RN;
Q546E/H/K/L/P/R;
M1043I/T/V;
H1047D/I/L/N/P/Q/R/T/Y; or
G1049A/C/D/R/S;
or a combination thereof.

11. The combination therapy for use of claim 9 or claim 10, wherein the mutation of PIK3CA comprises one mutation selected from the group consisting of E542K, E545K, Q546R, H1047L and H1047R, and a second mutation selected from the group consisting of E453Q/K, E726K and M1043L/I.

12. The combination therapy for use of claim 11, wherein the mutation of PIK3CA is selected from a double mutation selected from the group consisting of E542K + E453Q/K, E542K + E726K, E542K + M1043L/I; E545K + E453Q/K, E545K + E726K, E545K + M1043L/I; H1047R + E453Q/K, and H1047R + E726K.

13. The combination therapy for use of any one of claims 4-12, wherein the patient has not received prior chemotherapy before administration of the combination therapy.

14. The combination therapy for use of any one of claims 4-13, wherein the patient has not been previously treated with a PI3K, AKT, or mTOR inhibitor.

15. The combination therapy for use of any one of claims 4-14, wherein the patient has been previously treated with fulvestrant.

## Patentansprüche

1. Kombinationstherapie, umfassend GDC-9545 oder ein pharmazeutisch unbedenkliches Salz davon und GDC-0077 oder ein pharmazeutisch unbedenkliches Salz davon.

2. Kombinationstherapie nach Anspruch 1, wobei das GDC-9545 oder ein pharmazeutisch unbedenkliches Salz davon einmal täglich an den Tagen 1 - 28 eines ersten 28-Tage-Zyklus verabreicht wird und GDC-0077 oder ein pharmazeutisch unbedenkliches Salz davon einmal täglich an den Tagen 1 - 28 des ersten 28-Tage-Zyklus verabreicht wird.

3. Kombinationstherapie nach Anspruch 2, wobei GDC-0077 in einer Dosis von 9 mg verabreicht wird und GDC-9545 oder ein pharmazeutisch unbedenkliches Salz davon in einer Menge von 30 mg verabreicht wird.

4. Kombinationstherapie nach Anspruch 1 zur Verwendung in einem Verfahren zum Behandeln von Östrogenrezeptor-positivem und HER2-negativem lokal fortgeschrittenem Brustkrebs (laBC) oder metastatischem Brustkrebs (mBC) bei einem Patienten mit Rezeptorpositivem und HER2-negativem laBC oder mBC, wobei das Verfahren das Verabreichen einer Kombinationstherapie an den Patienten umfasst, die ein Dosierregime umfasst, das Folgendes umfasst:
i. Verabreichen von GDC-9545 oder eines pharmazeutisch unbedenklichen Salzes davon einmal täglich an den Tagen 1 - 28 eines ersten 28-Tage-Zyklus und
ii. Verabreichen von GDC-0077 einmal täglich an den Tagen 1 - 28 des ersten 28-Tage-Zyklus.

5. Kombinationstherapie zur Verwendung nach Anspruch 4, wobei GDC-0077 in einer Dosis von 9 mg verabreicht wird.

6. Kombinationstherapie zur Verwendung nach Anspruch 4 oder 5, wobei GDC-9545 oder ein pharmazeutisch unbedenkliches Salz davon in einer Menge von 30 mg verabreicht wird.

7. Kombinationstherapie zur Verwendung nach einem der Ansprüche 4-6, wobei sich der Patient in der Prämenopause befindet.

8. Kombinationstherapie zur Verwendung nach einem der Ansprüche 4-7, wobei der Patient auf die Gegenwart einer Mutation eines oder mehrerer von Östrogenrezeptor, Prostaglandinrezeptor oder Ki67 getestet wird.

9. Kombinationstherapie zur Verwendung nach einem der Ansprüche 4-8, wobei der Patient einen Tumor hat, der eine oder mehrere Mutation(en) von PIK3CA an Positionen, die R88Q; G106, K111, G118, N345, C420, E453, E542, E545, Q546, M1043, H1047 oder G1049 oder einer Kombination davon entsprechen, umfasst.

10. Kombinationstherapie zur Verwendung nach Anspruch 9, wobei die Mutation von PIK3CA
R88Q;
G106A/D/R/S/V;
K111N/R/E;
G118D;
N345D/H/I/K/S/T/Y;
C420R;
E453A/D/G/K/Q/V;
E542A/D/G/K/Q/R/V;
E545A/D/G/K/L/Q/R/V;
Q546E/H/K/L/P/R;
M1043I/T/V;
H1047D/I/L/N/P/Q/R/T/Y oder
G1049A/C/D/R/S
oder einer Kombination davon entspricht.

11. Kombinationstherapie zur Verwendung nach Anspruch 9 oder Anspruch 10, wobei die Mutation von PIK3CA eine Mutation, die aus der Gruppe ausgewählt ist, die aus E542K, E545K, Q546R, H1047L und H1047R besteht, und eine zweite Mutation, die aus der Gruppe ausgewählt ist, die aus E453Q/K, E726K und M1043L/I besteht, umfasst.

12. Kombinationstherapie zur Verwendung nach Anspruch 11, wobei die Mutation von PIK3CA aus einer Doppelmutation ausgewählt ist, die aus der Gruppe ausgewählt ist, die aus E542K + E453Q/K, E542K + E726K, E542K + M1043L/I; E545K + E453Q/K, E545K + E726K, E545K + M1043L/I; H1047R + E453Q/K und H1047R + E726K besteht.

13. Kombinationstherapie zur Verwendung nach einem der Ansprüche 4-12, wobei der Patient vor der Verabreichung der Kombinationstherapie keine vorausgehende Chemotherapie erhalten hat.

14. Kombinationstherapie zur Verwendung nach einem der Ansprüche 4-13, wobei der Patient zuvor nicht mit einem Pl3K-, AKT- oder mTOR-Inhibitor behandelt worden ist.

15. Kombinationstherapie zur Verwendung nach einem der Ansprüche 4-14, wobei der Patient zuvor mit Fulvestrant behandelt worden ist.

## Revendications

1. Polythérapie comprenant GDC-9545 ou un sel pharmaceutiquement acceptable de celui-ci et GDC-0077 ou un sel pharmaceutiquement acceptable de celui-ci.

2. Polythérapie selon la revendication 1, dans laquelle ledit GDC-9545 ou un sel pharmaceutiquement acceptable de celui-ci est administré une fois par jour aux jours 1 à 28 d'un premier cycle de 28 jours et GDC-0077 ou un sel pharmaceutiquement acceptable de celui-ci est administré une fois par jour aux jours 1 à 28 du premier cycle de 28 jours.

3. Polythérapie selon la revendication 2, dans laquelle GDC-0077 est administré à une dose de 9 mg et GDC-9545 ou un sel pharmaceutiquement acceptable de celui-ci est administré en une quantité de 30 mg.

4. Polythérapie selon la revendication 1 pour une utilisation dans une méthode de traitement d'un cancer du sein localement avancé (CSLA) ou d'un cancer du sein métastatique (CSM) positif aux récepteurs des œstrogènes et HER2-négatif chez une patiente ayant un CSLA ou un CSM positif aux récepteurs et HER2-négatif, la méthode comprenant l'administration à la patiente d'une polythérapie comprenant un schéma posologique comprenant :
i. l'administration de GDC-9545 ou d'un sel pharmaceutiquement acceptable de celui-ci une fois par jour aux jours 1 à 28 d'un premier cycle de 28 jours ; et
ii. l'administration de GDC-0077 une fois par jour aux jours 1 à 28 du premier cycle de 28 jours.

5. Polythérapie pour une utilisation selon la revendication 4, dans laquelle GDC-0077 est administré à une dose de 9 mg.

6. Polythérapie pour une utilisation selon la revendication 4 ou 5, dans laquelle GDC-9545 ou un sel pharmaceutiquement acceptable de celui-ci est administré en une quantité de 30 mg.

7. Polythérapie pour une utilisation selon l'une quelconque des revendications 4 à 6, dans laquelle la patiente est préménopausée.

8. Polythérapie pour une utilisation selon l'une quelconque des revendications 4 à 7, dans laquelle la patiente est testée pour la présence d'une mutation d'un ou plusieurs parmi le récepteur des œstrogènes, le récepteur des prostaglandines ou Ki67.

9. Polythérapie pour une utilisation selon l'une quelconque des revendications 4 à 8, dans laquelle la patiente a une tumeur comprenant une ou plusieurs mutations de PIK3CA aux positions correspondant à R88Q ; G106, K111, G118, N345, C420, E453, E542, E545, Q546, M1043, H1047 ou G1049, ou une combinaison de celles-ci.

10. Polythérapie pour une utilisation selon la revendication 9, dans laquelle la mutation de PIK3CA correspond à
R88Q ;
G106A/D/R/S/V ;
K111N/R/E ;
G118D ;
N345D/H/I/K/S/T/Y ;
C420R ;
E453A/D/G/K/Q/V ;
E542A/D/G/K/Q/R/V ;
E545A/D/G/K/L/Q/R/V ;
Q546E/H/K/L/P/R ;
M1043I/T/V;
H1047D/I/L/N/P/Q/R/T/Y ; ou
G1049A/C/D/R/S ;
ou une combinaison de celles-ci.

11. Polythérapie pour une utilisation selon la revendication 9 ou la revendication 10, dans laquelle la mutation de PIK3CA comprend une mutation choisie dans le groupe constitué par E542K, E545K, Q546R, H1047L et H1047R, et une seconde mutation choisie dans le groupe constitué par E453Q/K, E726K et M1043L/I.

12. Polythérapie pour une utilisation selon la revendication 11, dans laquelle la mutation de PIK3CA est choisie parmi une double mutation choisie dans le groupe constitué par E542K + E453Q/K, E542K + E726K, E542K + M1043L/I ; E545K + E453Q/K, E545K + E726K, E545K + M1043L/I ; H1047R + E453Q/K et H1047R + E726K.

13. Polythérapie pour une utilisation selon l'une quelconque des revendications 4 à 12, dans laquelle la patiente n'a pas reçu de chimiothérapie antérieure avant l'administration de la polythérapie.

14. Polythérapie pour une utilisation selon l'une quelconque des revendications 4 à 13, dans laquelle la patiente n'a pas été précédemment traitée avec un inhibiteur de PI3K, AKT ou mTOR.

15. Polythérapie pour une utilisation selon l'une quelconque des revendications 4 à 14, dans laquelle la patiente a précédemment été traitée avec du fulvestrant.
